# EUROPEAN PATENT SPECIFICATION

(11) **EP 4 048 522 B1**
(45) Date of publication and mention of the grant of the patent: **15.07.2026**
(21) Application number: 20808227.1
(22) Date of filing: 19.10.2020
(51) Int. Cl.: B32B 27/08, B32B 27/30, B32B 27/40, B32B 7/04, B32B 7/10, A61B 50/30, A61F 2/00

(54) **ORTHOPEDIC PACKAGING MATERIALS**
ORTHOPÄDISCHE VERPACKUNGSMATERIALIEN
LES MATÉRIAUX D'EMBALLAGE ORTHOPÉDIQUES

(30) Priority: 21.10.2019 US 201916658517
(43) Date of publication of application: 31.08.2022
(73) Proprietor: Covestro LLC, Pittsburgh, PA 15205 (US); Covestro Deutschland AG, 51373 Leverkusen (DE)
(72) Inventor: SCHULTZE, Dirk, 40764 Langenfeld (DE); STEBBINS, Lionel, Belchertown, Massachusetts 01007-9461 (US); KOSTHORST, Helge, 27374 Visselhövede (DE); KLEINHEIDER, Andre, 41352 Korschenbroich (DE)
(74) Representative: Levpat
(86) International application number: PCT/US2020/056260
(87) International publication number: WO 2021/080895

(56) References cited:
- WO-A1-2015/076780
- US-A1- 2014 053 908
- US-A1- 2017 239 922

## Description

### FIELD OF THE INVENTION

The present invention relates in general to packaging, and more specifically to packaging materials for sharp, abrasive articles, such as orthopedic implants.

### BACKGROUND OF THE INVENTION

Orthopedic implants have extremely sharp edges so as to be placed with minimum tolerances during surgery. Packaging of such sharp objects poses a challenge, as the sharp edges can generate abrasive dust from contacting and abrading the packaging materials. Surgical implants are typically packaged in a bag made of plastic for shipment from the manufacturer to the hospital for implantation.

This packaging bag must be durable enough on the outside to resist perforation, must exhibit appropriate barrier properties to regulate the transmission of oxygen and moisture, and must repel contaminants, such as bacteria. Tensile strength is necessary for burst, tear, and cut resistance across a wide range of temperatures. This allows for product resilience and is desirable for all applications where there is an abrasion risk.

Composite structures made from established materials are known in the art. These consist most commonly of PET-G for rigidity and stable dimensions, and a bag made from thermoplastic polyurethane providing superior surface abrasion stability.

Difficulties to be overcome in producing such packaging include, for example, the additional handling of a PET-G blister with a separate pouch, including an extended input of raw materials. This results in high manufacturing costs for the additional pouches as well as high costs for the manual packaging assembly. Difficulties can occur also on the user side in the hospital with nurses, who must handle multiple pieces during surgery / end-users. Nested blister packaging provides some benefit, but high processing costs remain. This results from requiring two different materials and two separate tools for thermoforming and deep-drawing. The same concerns likewise apply for PET-G blister, nested with alternative materials like silicone. In either case, the risk of the implant slipping and damaging the packaging remains.

Medical device packaging materials must also exhibit the appropriate barrier properties necessary to maintain the proper composition of fluids with which they interact, ensuring that oxygen, moisture and potential contaminants cannot penetrate through films or tubes. uch packaging must also possess the durability to withstand the rigors and fast pace of the medical environment, regardless of their rigidity. Finally, medical device packaging should demonstrate a clear history of biocompatibility.

Multilayer medical packaging made from elastomeric TPU films combined with PET-G films are available in the art and have been used for the packaging of orthopedic implants. Examples are e.g., grades such as Covestro's BAYFOL MA502, or MA504, all showing excellent performance. Such structured films provide higher strength and durability and have proven suitable for Form-Fill-Seal (FFS) packaging on highly automatized lines. WO2015076780A1 relates to packaging trays for medical devices.

Nevertheless, those skilled in the art continue to seek a solution providing better adhesion between the various layers. They are looking for the combination of the economics of cheaper materials with performance of technical superior materials.

A need continues to exist in the art for a way to overcome the adhesion deficits between incompatible polymers.

### SUMMARY OF THE INVENTION

Accordingly, the present invention provides a composite film structure having improved adhesion of thermoplastic polyurethane (TPU) to the various targeted thermoforming materials intended for laminating. Such laminates can subsequently be thermoformed into containers and trays to package abrasive, sharp medical articles such as orthopedic devices including bone implants.

These and other advantages and benefits of the present invention will be apparent from the Detailed Description of the Invention herein below.

### BRIEF DESCRIPTION OF THE FIGURES

The present invention will now be described for purposes of illustration and not limitation in conjunction with the figures, wherein:

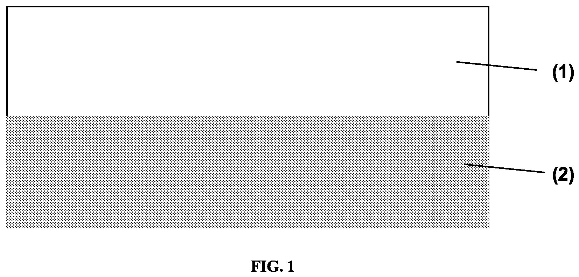

FIG. 1 shows a cross sectional view of the inventive packaging material.

### DETAILED DESCRIPTION OF THE INVENTION

The present invention will now be described for purposes of illustration and not limitation. Except in the operating examples, or where otherwise indicated, all numbers expressing quantities, percentages, and so forth in the specification are to be understood as being modified in all instances by the term "about."

Any numerical range recited in this specification is intended to include all sub-ranges of the same numerical precision subsumed within the recited range. For example, a range of "1.0 to 10.0" is intended to include all sub-ranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited in this specification is intended to include all lower numerical limitations subsumed therein and any minimum numerical limitation recited in this specification is intended to include all higher numerical limitations subsumed therein.

Reference throughout this specification to "various non-limiting embodiments," "certain embodiments," or the like, means that a particular feature or characteristic may be included in an embodiment. Thus, use of the phrase "in various non-limiting embodiments," "in certain embodiments," or the like, in this specification does not necessarily refer to a common embodiment, and may refer to different embodiments. Further, the particular features or characteristics may be combined in any suitable manner in one or more embodiments. Thus, the particular features or characteristics illustrated or described in connection with various or certain embodiments may be combined, in whole or in part, with the features or characteristics of one or more other embodiments without limitation. Such modifications and variations are intended to be included within the scope of the present specification.

The grammatical articles "a", "an", and "the", as used herein, are intended to include "at least one" or "one or more", unless otherwise indicated, even if "at least one" or "one or more" is expressly used in certain instances. Thus, these articles are used in this specification to refer to one or more than one (i.e., to "at least one") of the grammatical objects of the article. By way of example, and without limitation, "a component" means one or more components, and thus, possibly, more than one component is contemplated and may be employed or used in an implementation of the described embodiments. Further, the use of a singular noun includes the plural, and the use of a plural noun includes the singular, unless the context of the usage requires otherwise.

In one aspect, the present invention is directed to a packaging material comprising a first layer formed from a thermoplastic polyurethane (TPU) and a second layer formed from a heat-activated, olefin-based polymer coupling agent containing at least ethylene-vinyl acetate (EVA) copolymer and a second polar co-monomer, wherein the packaging material has a total thickness of between 2 mil (50 µm) and 24 mil (600 µm), the first layer has a thickness of between 0.8 mil (20 µm) and 16 mil (400 µm), and the second layer has a thickness of between 0.8 mil (20 µm) and 8 mil (200 µm).

Thermoplastic polyurethanes (TPUs) are well known in the art for their abrasion-stable characteristics. TPU is a robust material, and TPU products demonstrate high tear strength and resistance, durability even for soft grades, good chemical resistance, desired clarity, important abrasion resistance, and outstanding cold temperature performance. Solutions provided are for packaging including nested blister packaging, and composite blister packaging.

Because of the customizability of thermoplastic polyurethane (TPU), it is an ideal material for use in the construction of various medical devices, such as tubes and fluid storage bags. The safety of TPU products with regard to their use on or within the body is important, as medical products must often transmit drugs, blood or other fluids, and thus materials used in their development must not exude any harmful substances that could leach into these fluids and compromise fluid integrity. The combination of materials does not have to happen in one step. Lamination of materials is a preferred way to obtain a thermos-formable product for subsequent thermo-forming or FFS packaging.

New packaging designs may combine the mechanical puncture, and abrasion stability of TPU-elastomers with the thermoforming properties of rigid polymers, e.g. polyethylene terephthalate glycol (PETG), cyclo-olefin copolymers (COC), or polyolefin, e.g. cross linked polyethylene (PE) foam. This may be done by forming laminates of separate materials and customizing those laminates with the thermoforming process. If bonded in a reliable consistent way, these layers may be formed jointly into specific shapes addressing the needs for securely holding orthopedic medical implants in place. Such user-friendly designs help to avoid a more complex packaging structure consisting of separate sleeves, or pouches of abrasion stable layers and a rigid thermoformed outer packaging box.

Thermoplastic polyurethane (TPU) formulations can be tailored to the usage requirements of the medical industry. Because there are no plasticizers involved in TPU production, harmful byproducts are eliminated. TPUs are known for their reliably in the medical field, and their formulations have long been certified as biocompatible.

Ease of sealing is necessary to ensure the longevity of medical products. Such sealing techniques as ultrasound and solvent welding can provide a more efficient and consistent weld quality than other conventional methods. The use of these sealing techniques with thermoplastic polyurethane (TPUs) is well known to those in the art.

As shown in FIG. 1, the packaging material according to the invention comprises a first layer 1 made of a thermoplastic polyurethane (TPU) and a second layer 2 comprising a modified ethylene-vinyl acetate (EVA).

In certain embodiments, the second layer 2 comprises EVA copolymers modified by TPU, made by blending both TPU and EVA, having improved bonding to different polymers. This blend approach provides excellent bonding characteristics to both TPU and to rigid thermoforming polymers. In these embodiments, packaging materials according to the invention contain at least one layer of a TPU/EVA blend.

The second layer 2 also contains polar co-monomers, such as disclosed in U.S. Pat. No. 5,593,775. Ethylene is preferred as a co-monomer. In the packaging material according to the invention, at least two different polar, oxygen-containing olefin copolymers A and B are mixed and melted together under sufficient shear. The total proportion of polar oxygen-containing co-monomer units in various embodiments is between 18 wt. % and 40 wt. %, with reference to the total weight of polymer resin used. The olefin co-monomer A should be present in larger amounts, with a total proportion of at least 60% of the total weight of co-monomer units. In certain embodiments, the co-monomers used for the polar olefin copolymers A and B are methacrylic acid and/or its salts and/or esters and/or acrylic acid and/or its salts and/or its esters and/or vinyl acetate, in the unsaponified and/or saponified form.

In some embodiments, the TPU ether-based films are preferred over TPU ester-based films due to the former's improved hydrolysis resistance.

Additionally, the co-extrusion manufacture of a film comprising a plain TPU layer facing one surface and at least one TPU/EVA layer facing the other surface for bonding purposes, improves the performance of the inventive packaging material. The co-extrusion process provides options for further bridging by combining with additional layers. Such a coextruded film is in particular suitable for flame bonding to crosslinked polyethylene (PE) foam. Surface improved PE foam, as known in the art, is suitable for packaging medical devices and in particular, for medical device packaging intended for surgical implants.

Adhesive-modified EVA provides improved adhesion properties between TPU and structured olefin surfaces. Adhesion properties may be introduced by either inserting an adhesive layer between the TPU and the olefin, or by blending TPU with the olefin. In certain embodiments, this may be done by providing a bridging layer combining the compatibility through blending TPU with EVA and having a TPU surface layer.

Suitable olefinic materials for use in the present invention include, but are not limited to, polyethylene (e.g., high-density HDPE, low-density LDPE, and linear low-density LLDPE), polypropylene, polymethylpentene, polyisobutylene, polybutylene, and ethylene propylene diene monomer (EPDM) rubber.

Adhesive properties for the second layer 2 show a most suitable performance for TPU/EVA blend ratio between 0.3 and 3.0 mass-percent. In certain embodiments, the blend ratio between TPU and modified EVA is between 0.7 and 1.5 mass-percent.

TPU and EVA are accessible to thermoplastic techniques due to a joint processing window which makes them suitable partners for enhancing adhesion properties. Use of the joint thermoplastic window may be made in the film extrusion process, allowing for co-extrusion of various layers.

In some embodiments, EVA-maleic anhydride (MSA) terpolymers may be selected. Those polymers may be randomly copolymerized, or in some embodiments, MSA may be grafted to an EVA backbone. Certain embodiments of the inventive packaging material contain at least two layers as follows: one layer of TPU **1** providing abrasion stability, and one layer of a TPU/EVA blend **2** providing adhesion.

In certain embodiments, the coupling substance resins being used for the second layer **2,** contain ethylene, vinyl acetate, and maleic acid anhydride as co-monomers, with the total amount of maleic acid anhydride not exceeding 5000 ppm.

The first layer **1** of the packaging material according to the invention comprises at least one TPU elastomer, preferably a linear TPU elastomer, of which the longer chain diol component is a polyester or polyether, and a Shore hardness of preferably 75 - 95 A, measured according to ASTM D 2240. TPU ether-based films are preferred over TPU ester-based films due to the former's improved hydrolysis resistance.

Suitable thermoplastic polyurethanes are available from a variety of commercial suppliers under the trade names: DESMOPAN, ELASTOLLAN, ESTANE, MORTHANE AND TEXIN.

Packaging materials according to various embodiments of the invention may contain within the first layer **1,** additional processing additives, such as the following:
a) anti-blocking agents, inorganic or organic spacers,
b) slip or separation aids, typically surface active,
c) pigments or fillers and
d) stabilizers.
The total of additives a, b, c, and d in selected embodiments is between 0% and 30%, in certain embodiments, the total is between >0% and 30%.

In various embodiments, films have a total thickness between 2 mil (50 µm) and 24 mil (600 µm). In those embodiments, the layer thickness for the first (TPU) layer **1** is between 0.8 mil (20 µm) and 16 mil (400 µm), and the gauge of the second (blend) layer **2** is between 0.8 mil (20 µm) and 8 mil (200 µm).

To manufacture multilayer structures such as packaging material, known thermal forming procedures for the processing of plastic materials may be used. In particular, co-extrusion techniques are preferred for the better levels of bond strength among the layers at temperatures of from 160°C to 200°C. Blown film co-extrusion is a particularly preferred method to produce packaging material according to the invention.

During manufacturing of the inventive packaging material, films can be subjected to surface treatment, such that at least one of the outer layers of the packaging material is subjected to a chemical or physical treatment.

Packaging materials according to the invention may be used in the manufacture of containers (e.g., bags, trays, etc.) for sharp, abrasive articles by lamination to rigid plastic materials followed by thermoforming. They provide the advantage of being accessible by various thermal and solvent-based sealing methods. The packaging materials according to the present invention are particularly advantageous in the packaging of sharp, abrasive articles such as medical, in particular, orthopedic implants.

### EXAMPLES

The non-limiting and non-exhaustive examples that follow are intended to further describe various non-limiting and non-exhaustive embodiments without restricting the scope of the embodiments described in this specification. All quantities given in "parts" and "percents" are understood to be by weight, unless otherwise indicated.

The following examples and comparison examples were manufactured by blown film extrusion. The extrusion process suitable for processing thermoplastic materials has been described among others by Wortberg, Mahlke and Effen in: Kunststoffe, 84 (1994) 1131-1138, by Pearson in: Mechanics of Polymer Processing, Elsevier Publishers, New York, 1985, and company Davis-Standard in: Paper, Film & Foil Converter 64 (1990) pp 84 - 90. Blown film tools to shape the melt into film shape are explained by Rauwendaal in: Polymer Extrusion, Hanser Publishers, New York 1986, and Michaeli in: Extrusions-Werkzeuge, Hanser Verlag, Munich 1991.

### Example 1

Using a two-layer blown film die, a coextruded film was manufactured comprising a first layer formed from a TPU-ether of Shore-A-hardness 89, measured according to ASTM D2240, corresponding to a hardness of 36 Shore-D. The TPU-ether had a melt flow index (MFI) of 25 g/10 min @ 190°C/21.6 kg according to ISO 1133-1, a specific gravity of 1.12 g/cm³ according to ISO 1183-1, and a thermomechanical analysis (TMA) onset temperature of 165°C. This 100 µm layer contained the processing additives 2.5% diatomaceous earth and 0.3% amide wax. All ingredients for this first layer were processed in a single extruder.

The second layer was an ethylene-vinyl-acetate copolymer with a melt flow index (MFI) of 3.5 g/10 min @ 230°C/2.16 kg according to ISO 1133-1, and a specific gravity of 0.94 g/cm³ according to ISO 1183-1, which was grafted with maleic anhydride. The vinyl-acetate (VA) content of the resin was 28 mass-percent and maleic anhydride was below 4000 ppm. The Shore hardness was at 80A / 27 D according to ASTM D2240. The TMA onset temperature was 75°C. The second layer had a thickness of 50 µm.

The extrusion tools were set to temperatures between 160°C and 200°C. The two melt streams were joined in a two-layer blown film die at a processing temperature of 195°C and ejected through a circular die with a diameter of 130 mm. The circular melt was cooled by blowing chilled air against it. The film subsequently collapsed, was laid flat, separated, and was wound up.

### Example 2

Using a two-layer blown film die, a coextruded film was manufactured from a first layer formed from a TPU-ether of Shore-A-hardness 89, measured according to ASTM D2240, corresponding to a hardness of 36 Shore-D. The TPU-ether had a melt flow index (MFI) of 25 g/10 min @ 190°C/21.6 kg according to ISO 1133-1, a specific gravity of 1.12 g/cm³ according to ISO 1183-1, and a TMA onset temperature of 165°C. This 90 µm layer contained the processing additives 2.5% diatomaceous earth and 0.3% amide wax. All ingredients for this first layer were processed in a single extruder.

The second layer was a blend of 50% by weight of TPU-ether with a melt flow index (MFI) of 25 @ 190°C/21.6 kg according to ISO 1133-1, a specific gravity of 1.12 g/cm³ according to ISO 1183-1 at a Shore A hardness of A 89 / D36 according to ASTM D2240, and a TMA onset of 165°C. 50% by weight was a grafted ethylene-vinyl-acetate/maleic anhydride copolymer with a melt flow index (MFI) of 3.5 g/10 min @ 230°C/2.16 kg according to ISO 1133-1 and a specific gravity of 0.94 g/cm³ according to ISO 1183-1. The vinyl-acetate content of the resin was at 28 mass-percent and maleic anhydride was below 4000 ppm. The Shore hardness was at 80A / 27D according to ASTM D2240. The TMA onset temperature was at 75°C. The second layer had a thickness of 60 µm.

The extrusion tools were set to temperatures between 160°C and 200°C. The two melt streams were joined in a two-layer blown film die at a processing temperature of 195°C and ejected through a circular die with a diameter of 130 mm. The circular melt was cooled by blowing chilled air against it. The film subsequently collapsed, was laid flat, separated, and was wound up.

### Comparative Example 1

Using a mono layer blown film die, a film was manufactured from a TPU-ether of Shore-A-hardness 89, measured according to ASTM D2240, corresponding to a hardness of 36 Shore-D. The TPU-ether had a melt flow index (MFI) of 25 g/10 min @ 190°C/21.6 kg according to ISO 1133-1, a specific gravity of 1.12 g/cm³ according to ISO 1183-1, and a TMA onset temperature of 165°C. This 150 µm layer contained the processing additives 2.5% diatomaceous earth and 0.3% amide wax. All ingredients for this layer were processed in a single extruder.

The extruder was operated at temperatures between 160°C and 200°C. The melt streams were inserted into a blown film die at a processing temperature of 195°C and ejected through a lip set of 130 mm diameter. The circular melt was cooled by blowing chilled air against it. The film subsequently collapsed, was laid flat, separated, and was wound up.

### Comparative Example 2

Using a two-layer blown film die, a coextruded film was manufactured consisting of a layer formed from a TPU-ester of a Shore-A-hardness 90, measured according to ASTM D2240, corresponding to a hardness of 40 Shore-D. The TPU ester had a melt flow index (MFI) of 60 g/10 min @ 190°C/21.6 kg according to ISO 1133-1, a specific gravity of 1.22 g/cm³ according to ISO 1183-1, and a TMA onset temperature of 170°C. This 30 µm layer contained the processing additives 5% diatomaceous earth and 1% amide wax. All ingredients for this layer were processed in a single extruder.

LA second layer was made essentially of a linear hydroxyl polyester polyurethane. This flexible hot melt adhesive polyurethane with a high rate of crystallization has typically outstanding adhesion on a large number of materials. Extruded flat products made from this raw material are known to be of high quality. This TPU-ester had a viscosity of 1200 m Pas measured according to ISO 3219. a specific gravity of 1.16 g/cm³ according to ISO 1183-1, a Shore A hardness of A 94 / D 45 according to ASTM D2240, and a TMA onset temperature of 55 °C. The second layer had a thickness of 20 µm.

The extrusion tools were set to temperatures between 130°C and 180°C. The melt streams were joined in a multilayer blown film die at a processing temperature of 180°C and ejected through a set of circular lips with a diameter of 300 mm. The circular melt was cooled by blowing chilled air against it. The film subsequently collapsed, was laid flat, separated, and was wound up.

### Comparative Example 3

Using a mono layer blown film die, a film was manufactured from a TPU-ester of Shore-A-hardness 90, measured according to ASTM D2240, corresponding to a hardness of 39 Shore-D. The TPU-ester had a melt flow index (MFI) of 16 g/10 min @ 190°C/8.7 kg according to ISO 1133-1, a specific gravity of 1.19 g/cm³ according to ISO 1183-1, and a TMA onset temperature of 162°C. This 130 µm layer contained the processing additives 1.5% diatomaceous earth and 0.4% amide wax. All ingredients for this layer were processed in a single extruder.

The extruder was operated at temperatures between 160°C and 190°C. The melt stream was inserted into a blown film die at a processing temperature of 185°C and ejected through a lip set of 130 mm diameter. The circular melt was cooled by blowing chilled air against it. The film subsequently collapsed, was laid flat, separated, and was wound up.

Evaluation of the properties of the inventive samples and comparative samples considered adhesion to polyethylene directly and during thermoforming as important characteristics. The bond strength to an olefin layer was tested among the films prepared as samples and comparative examples.

Lamination of films made as samples and as comparative examples was carried out using a HOTRONIX laminator. The films were laminated against a polyethylene (PE) film of 0.0045 in. / 115 µm thickness. The PE had a melt flow index (MFI) of 0.4 g/10 min ISO 1133-1 @ 190°C, 2.16 kg, a density of 0.92g/cm³ ISO 1183-1, and a hardness of 45D/94A Shore. The PE film had a TMA onset temperature of 103°C. Lamination was carried out at 150°C for 10 seconds.

The visual descriptions of manual separation, as well as the pull test results from a testing device were determined.

For the Pull Test, 1 in. / 25.4 mm wide sample strips were cut out of the laminates using a parallel blade sample cutter. The separation force was determined using a regular samples clamps and load cell of a tensile testing set up.

To obtain thermoforming results, blanks of about 125 mm diameter were cut of the laminates and heated using a MINISTAR S thermal-forming device. They were heated for about 20 seconds in the infra-red (IR) field, and then drawn over a cylindrical round bottomed shape (height of 75 mm and a diameter of 75 mm).

Table I summarizes the results of the above-detailed examples.

**Table I**

| **Property** | **Example 1** | **Example 2** | **Comparative Example 1** | **Comparative Example 2** | **Comparative Example 3** |
|---|---|---|---|---|---|
| | EVA layer facing PE | TPU / EVA blend layer facing PE | | Hot melt adhesive polyurethane layer facing PE | |
| Delamination - Description | Film stretching failure - very good bond | Film stretching failure - good bond | Bond failure - very weak bond | Bond failure - very weak bond | Bond failure - very weak bond |
| Pull Test - Peel Strength g/in. (gf) | 825 | 292 | 10 | 8 | 7 |
| Thermoforming - Visual description | Consistent, visually uniform, transparent | Consistent, hazy, cloudy | Consistent, visually uniform, hazy | Consistent, visually uniform, hazy | Consistent, visually uniform, transparent |
| Thermoformed parts | No layer separation | No layer separation | Layer separation | Layer separation | Layer separation |

As is apparent by reference to Table I, the films made according to the present invention are superior to the comparative films.

## Claims

1. A packaging material comprising
a first layer formed from a thermoplastic polyurethane and
a second layer formed from a heat-activated, olefin-based polymer coupling agent containing an
ethylene-vinyl acetate copolymer and a second polar co-monomer,
wherein the packaging material has a total thickness of between 50 µm and 600 µm, the first layer has a thickness of between 20 µm and 400 µm, and the second layer has a thickness of between 20 µm and 200 µm.

2. The packaging material according to claim 1, wherein the first layer comprises thermoplastic polyurethane-ether.

3. The packaging material according to claim 1 or 2, wherein the first layer has a Shore hardness, measured according to ASTM D 2240, of 75 - 95 A.

4. The packaging material according to claim 1, 2 or 3, wherein the second layer is a blend comprising thermoplastic polyurethane and ethylene-vinyl acetate copolymer.

5. The packaging material according to any of claims 1 to 4, wherein the ethylene-vinyl acetate copolymer contains at least 15% vinyl acetate.

6. The packaging material according to any of claims 1 to 5, wherein the olefin-based polymer coupling agent contains less than 5000 ppm of maleic anhydride.

7. The packaging material according to any of claims 1 to 6, wherein the second layer comprises a blend of thermoplastic polyurethane and a modified ethylene-vinyl acetate at a ratio of between 0.3 mass-percent and 3.0 mass-percent.

8. The packaging material according to any of claims 1 to 7, wherein the second layer comprises a blend of thermoplastic polyurethane and a modified ethylene-vinyl acetate at a ratio of between 0.7 mass-percent and 1.5 mass-percent.

9. The packaging material according to any of claims 1 to 8, wherein the second polar co-monomer comprises one monomer selected from the group consisting of vinyl acetate, acrylic acid, acrylic acid esters, acrylic acid salts, methacrylic acid, methacrylic acid esters, and methacrylic acid salts.

10. The packaging material according to any of claims 1 to 9, wherein between 0% and 30% of an additive selected from the group consisting of anti-blocking agents, inorganic or organic spacers, slip or separation aids, pigments, fillers, and stabilizers, is added to the first layer.

11. The packaging material according to any of claims 1 to 10, wherein the packaging material is produced in a co-extrusion process at a temperature of from 160°C to 200°C.

12. The packaging material according to any of claims 1 to 11, wherein the packaging material is produced in a blown film co-extrusion process at a temperature of from 160°C to 200°C.

13. The packaging material according to any of claims 1 to 12, wherein at least one layer is subjected to a physical or chemical surface treatment.

14. One of a container and a tray comprising thermoformed packaging material according to claim 1.

15. The one of a container and a tray according to claim 14, wherein the packaging material is laminated to a polyethylene or a cross-linked polyethylene by one selected from the group consisting of thermo-lamination and flame bonding.

## Patentansprüche

1. Verpackungsmaterial umfassend
eine erste Schicht, die aus einem thermoplastischen Polyurethan gebildet ist, und
eine zweite Schicht, die aus einem wärmeaktivierten Polymerkupplungsmittel auf Olefinbasis gebildet ist, das ein Ethylen-Vinylacetat-Copolymer und ein zweites polares Co-Monomer enthält,
wobei das Verpackungsmaterial eine Gesamtdicke von zwischen 50 µm und 600 µm aufweist, die erste Schicht eine Dicke von zwischen 20 µm und 400 µm aufweist und die zweite Schicht eine Dicke von zwischen 20 µm und 200 µm aufweist.

2. Verpackungsmaterial nach Anspruch 1, wobei die erste Schicht thermoplastischen Polyurethanether umfasst.

3. Verpackungsmaterial nach Anspruch 1 oder 2, wobei die erste Schicht eine Shore-Härte, gemessen nach ASTM D 2240, von 75-95 A aufweist.

4. Verpackungsmaterial nach Anspruch 1, 2 oder 3, wobei die zweite Schicht ein Gemisch ist, das thermoplastisches Polyurethan und Ethylen-Vinylacetat-Copolymer umfasst.

5. Verpackungsmaterial nach einem der Ansprüche 1 bis 4, wobei das Ethylen-Vinylacetat-Copolymer wenigstens 15 % Vinylacetat enthält.

6. Verpackungsmaterial nach einem der Ansprüche 1 bis 5, wobei das Polymerkupplungsmittel auf Olefinbasis weniger als 5000 ppm Maleinsäureanhydrid enthält.

7. Verpackungsmaterial nach einem der Ansprüche 1 bis 6, wobei die zweite Schicht ein Gemisch aus thermoplastischem Polyurethan und einem modifizierten Ethylen-Vinylacetat in einem Verhältnis zwischen 0,3 Massenprozent und 3,0 Massenprozent umfasst.

8. Verpackungsmaterial nach einem der Ansprüche 1 bis 7, wobei die zweite Schicht ein Gemisch aus thermoplastischem Polyurethan und einem modifizierten Ethylen-Vinylacetat in einem Verhältnis zwischen 0,7 Massenprozent und 1,5 Massenprozent umfasst.

9. Verpackungsmaterial nach einem der Ansprüche 1 bis 8, wobei das zweite polare Co-Monomer ein Monomer ausgewählt aus der Gruppe bestehend aus Vinylacetat, Acrylsäure, Acrylsäureestern, Acrylsäuresalzen, Methacrylsäure, Methacrylsäureestern und Methacrylsäuresalzen umfasst.

10. Verpackungsmaterial nach einem der Ansprüche 1 bis 9, wobei der ersten Schicht zwischen 0 % und 30 % an einem Additiv ausgewählt aus der Gruppe bestehend aus Antiblockiermitteln, anorganischen oder organischen Abstandshaltern, Gleit- oder Trennhilfsmitteln, Pigmenten, Füllstoffen und Stabilisatoren zugesetzt ist.

11. Verpackungsmaterial nach einem der Ansprüche 1 bis 10, wobei das Verpackungsmaterial durch ein Coextrusionsverfahren bei einer Temperatur von 160 °C bis 200 °C hergestellt ist.

12. Verpackungsmaterial nach einem der Ansprüche 1 bis 11, wobei das Verpackungsmaterial durch ein Blasfolien-Coextrusionsverfahren bei einer Temperatur von 160 °C bis 200 °C hergestellt ist.

13. Verpackungsmaterial nach einem der Ansprüche 1 bis 12, wobei wenigstens eine Schicht einer physikalischen oder chemischen Oberflächenbehandlung unterzogen ist.

14. Eines von einem Behälter und einer Schale, umfassend thermogeformtes Verpackungsmaterial nach Anspruch 1.

15. Eines von einem Behälter und einer Schale nach Anspruch 14, wobei das Verpackungsmaterial durch eines ausgewählt aus der Gruppe bestehend aus Thermolaminierung und Flammbindung auf ein Polyethylen oder ein vernetztes Polyethylen laminiert ist.

## Revendications

1. Matériau d'emballage comprenant
une première couche formée à partir d'un polyuréthane thermoplastique et
une deuxième couche formée à partir d'un agent de couplage de polymère à base d'oléfine, activé thermiquement contenant un copolymère d'éthylène-acétate de vinyle et un deuxième comonomère polaire,
dans lequel le matériau d'emballage a une épaisseur totale comprise entre 50 µm et 600 µm, la première couche a une épaisseur comprise entre 20 µm et 400 µm, et la deuxième couche a une épaisseur comprise entre 20 µm et 200 µm.

2. Matériau d'emballage selon la revendication 1, dans lequel la première couche comprend un polyuréthane-éther thermoplastique.

3. Matériau d'emballage selon la revendication 1 ou 2, dans lequel la première couche a une dureté Shore, mesurée selon la norme ASTM D 2240, de 75 à 95 A.

4. Matériau d'emballage selon la revendication 1, 2 ou 3, dans lequel la deuxième couche est un mélange comprenant un polyuréthane thermoplastique et un copolymère éthylène-acétate de vinyle.

5. Matériau d'emballage selon l'une quelconque des revendications 1 à 4, dans lequel le copolymère éthylène-acétate de vinyle contient au moins 15 % d'acétate de vinyle.

6. Matériau d'emballage selon l'une quelconque des revendications 1 à 5, dans lequel l'agent de couplage de polymère à base d'oléfine contient moins de 5 000 ppm d'anhydride maléique.

7. Matériau d'emballage selon l'une quelconque des revendications 1 à 6, dans lequel la deuxième couche comprend un mélange de polyuréthane thermoplastique et d'un éthylène-acétate de vinyle modifié dans un rapport compris entre 0,3 pour cent en masse et 3,0 pour cent en masse.

8. Matériau d'emballage selon l'une quelconque des revendications 1 à 7, dans lequel la deuxième couche comprend un mélange de polyuréthane thermoplastique et d'un éthylène-acétate de vinyle modifié dans un rapport compris entre 0,7 pour cent en masse et 1,5 pour cent en masse.

9. Matériau d'emballage selon l'une quelconque des revendications 1 à 8, dans lequel le deuxième comonomère polaire comprend un monomère choisi dans le groupe constitué par l'acétate de vinyle, l'acide acrylique, les esters d'acide acrylique, les sels d'acide acrylique, l'acide méthacrylique, les esters d'acide méthacrylique et les sels d'acide méthacrylique.

10. Matériau d'emballage selon l'une quelconque des revendications 1 à 9, dans lequel entre 0 % et 30 % d'un additif choisi dans le groupe constitué par les agents anti-blocage, les espaceurs inorganiques ou organiques, les auxiliaires de glissement ou de séparation, les pigments, les charges et les stabilisants, est ajouté à la première couche.

11. Matériau d'emballage selon l'une quelconque des revendications 1 à 10, dans lequel le matériau d'emballage est produit dans un procédé de co-extrusion à une température de 160 °C à 200 °C.

12. Matériau d'emballage selon l'une quelconque des revendications 1 à 11, dans lequel le matériau d'emballage est produit dans un procédé de co-extrusion de film soufflé à une température de 160 °C à 200 °C.

13. Matériau d'emballage selon l'une quelconque des revendications 1 à 12, dans lequel au moins une couche est soumise à un traitement de surface physique ou chimique.

14. Récipient ou plateau comprenant un matériau d'emballage thermoformé selon la revendication 1.

15. Récipient ou plateau selon la revendication 14, dans lequel le matériau d'emballage est stratifié sur un polyéthylène ou un polyéthylène réticulé par l'un choisi dans le groupe constitué par la thermo-stratification et le collage à la flamme.
